# EUROPEAN PATENT SPECIFICATION

(11) **EP 1 688 502 B1**
(45) Date of publication and mention of the grant of the patent: **14.04.2010**
(21) Application number: 04791394.2
(22) Date of filing: 29.10.2004
(51) Int. Cl.: C12P 7/66

(54) **MICROBIOLOGICAL METHOD FOR THE PREPARATION OF MENADIONE**
MIKROBIOLOGISCHES VERFAHREN ZUR HERSTELLUNG VON MENADION
PROCEDE MICROBIOLOGIQUE DE PREPARATION DE MENADIONE

(30) Priority: 31.10.2003 ES 200302622
(43) Date of publication of application: 09.08.2006
(73) Proprietor: Institut Univ de Ciència i Tecnologia, 08100 Mollet del Vallès (ES)
(72) Inventor: ESTÉVEZ COMPANY, Carles, E-08100 Mollet del Vallès (ES); SOLDEVILA FÀBREGA, Andreu, E-08100 Mollet del Vallès (ES); CASTELLS BOLIART, Josep, E-08100 Mollet del Vallès (ES); MONTILLA ARÉVALO, Rafael, E-08100 Mollet del Vallès (ES)
(74) Representative: ZBM Patents
(86) International application number: PCT/ES2004/000475
(87) International publication number: WO 2005/042755

(56) References cited:
- JP-A- 4 312 542
- JP-A- 6 022 775
- JP-A- 9 107 983
- ESCAMILLA J E ET AL: "Role of menaquinone in inactivation and activation of the Bacillus cereus forespore respiratory system" JOURNAL OF BACTERIOLOGY, vol. 170, no. 12, December 1988 (1988-12), pages 5908-5912,

## Description

This invention relates to the field of industrial microbiology, and specifically to a new preparation process of a low molecular weight compound using microorganisms.

### BACKGROUND ART

Menadione or 2-methyl-1,4-naphtalenedione is also known as vitamin K₃ or synthetic K₃ vitamin and it has the accompanying chemical formula (I). Menadione plays an essential role in blood coagulation, acting in the synthesis of protrombine and blood coagulation factors VII, IX and X. It also acts in the conversion of hepatic glucose into glycogen. Apparently, it has an important role in the synthesis of human bones, reason for which it is also used for the prevention of osteoporosis. The necessary menadione daily income ranges from 5 µg (children under 3 years old) to 80 µg (adults) which is usually covered by a diet rich in vegetables. Deficiency in menadione, mainly due to intestinal alterations, may lead to coagulation and vascular problems. For these reasons, menadione is considered an active agent produced and used worldwide, both in animal and human therapy.

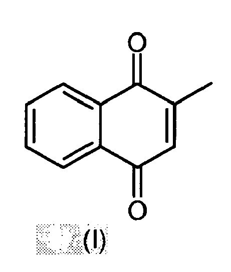

At industrial level, menadione is mainly synthesized by an extremely pollutant oxidative process, which includes very dangerous and toxic chemical substances. This process involves the oxidation of 2-methylnaphtalene (II-1) with chromic acid or derivatives (CrO₃ or Na₂Cr₂O₇, cf. US 2.402.226). Numerous processes for the manufacture of menadione have been suggested trying to improve the current industrial synthesis. One of them is based on the oxidation with hydrogen peroxide (cf. US 2.373.003), but it has been proved not feasible at industrial level. Another process is based on the oxidation of 2-methylnaphtalene with sodium dichromate, sulfuric acid, acetic acid and pyridine (cf. US 3.751.437), which also are highly contaminant chemicals.

One of the main problems of synthetic pathways known in the art is the concomitant production of a regioisomer (III) which does not have the pharmacological activity of menadione. The presence of this subproduct makes more difficult and expensive the steps of purification of menadione. There have been several other attempts trying to improve the above-mentioned pollutant processes, but their scale-up to industrial level have not been successful. Examples of these alternative processes involve the oxidation in a multitubular reactor with vanadium oxide, potassium pyrosulfate and potassium sulfate, or the oxidation by potassium hydrogensulfate and a manganese-containing porphyrine.

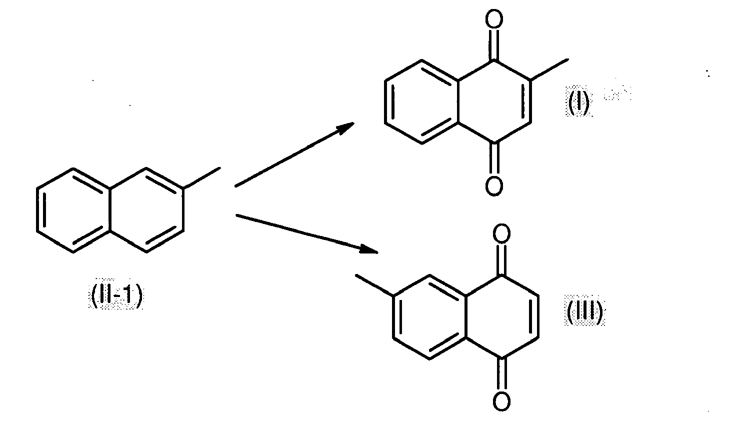

In order to solve the pollution problems of chemical preparation processes, a bio-based approach has been reported which involves the use of a bacterium from the genus Rhodococcus (cf. JP 6022775), although it has not been fully industrialized yet. Thus, it seems highly desirable to provide alternative industrial microbiological processes for the preparation of menadione, which are both non-pollutant and regioselective.

Escamilla et al., Journal of Bacteriology, Vol. 170, No. 12, December 1988, pages 5908-5912, is a study of the role of menaquinone in the inactivation and activation of the Bacillus cereus forespore respiratory system. It is disclosed that incubation of Bacillus cereus membranes with menadiol leads to oxidation of menadiol to menadione.

### SUMMARY OF THE INVENTION

Inventors have surprisingly found an alternative process for the preparation of menadione. The process involves the elimination of acid waste and toxic chemicals, so it is environmentally benign, safe and economical. An additional advantage is that the use of microorganisms as biocatalysts allows a selective process in which no isomer is substantially produced.

Thus, the invention provides a preparation process of menadione comprising cultivating bacteria Bacillus cereus in a suitable culture medium comprising one or more substrates selected from the group consisting of 2-methylnaphtalene, 2-methyl-1-naphtol, and 3-methyl-1-naphtol.

The possible substrates are II-1, II-2 and II-3. In a preferred embodiment, the substrate used is 2-methylnaphtalene (II-1), 2-methyl-1-naphtol (II-2) or mixtures thereof. In a more preferred embodiment, the substrate is 2-methylnaphtalene (II-1).

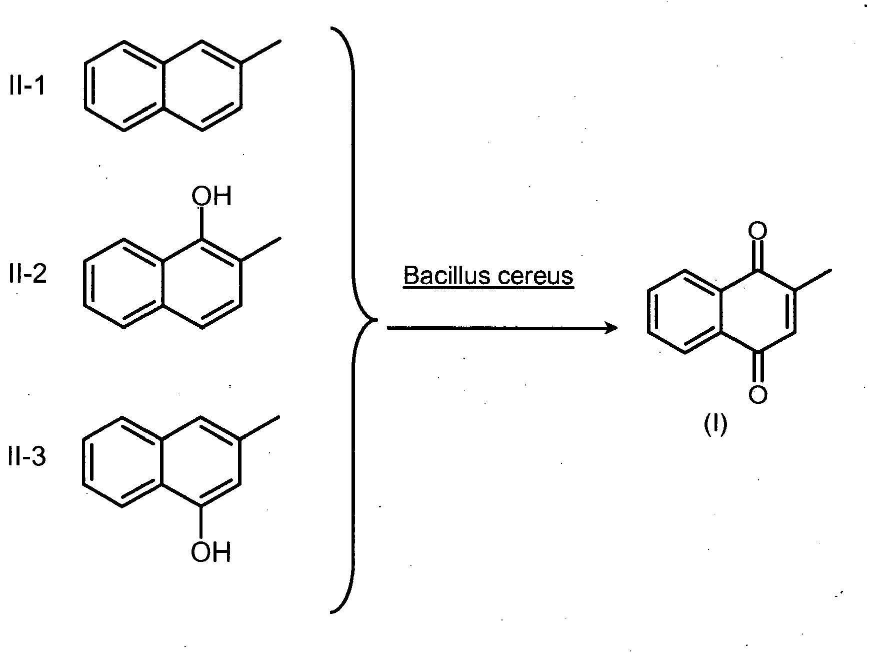

In a preferred embodiment of the invention the bacteria Bacillus cereus are of biotype I, being bacterial cells disrupted or whole. In particular embodiments the bacterial cells have been precultivated in a culture medium comprising a succinate alkaline salt. Furthermore, in other embodiments of the invention, the culture medium used for preparing menadione comprises a linear saturated hydrocarbon of 10 to 18 carbon atoms, particularly hexadecane, in a concentration in the culture medium from 25 mg/l to 5 g/I.

Throughout the description and claims the word "comprise" and its variations are not intended to exclude other technical features, additives, components, or steps. The following particular embodiments are provided by way of illustration, and are not intended to be limiting of the present invention.

### DETAILED DESCRIPTION OF PARTICULAR EMBODIMENTS

### Assay 1: Preparation of menadione with Bacillus cereus DSM 31

Preliminary growth of the microorganism: Precultures of Bacillus cereus DSM 31 (accession number for the deposit in the Deutsche Sammlung von Mikroorganismen und Zellkulturen GmbH) were grown at pH 7 in trypticase soy medium. One percent of the culture was passed into minimal medium containing yeast extract (0.4 g/l), Na₂HPO₄ (4.4 g/l), KH₂PO₄ (2.45 g/l), (NH₄)₂SO₄ (1.98-g/l); MgSO₄ (0.24 g/l), MnCl₂ (0.025 g/l), FeSO₄ (0.015 g/l) and sodium succinate (5.5 g/l). It was incubated at 35 ºC with vigorous shaking (200 rpm) until early stationary phase. Cells were harvested using a centrifugation step of 20 min at 4500 rpm, and they were washed in phosphate buffer.

Menadione preparation: Cells were passed into minimal medium containing yeast extract (0.4 g/l), Na₂HPO₄ (4.4 g/l), KH₂PO₄ (2.45 g/l), (NH₄)₂SO₄(1.98 g/l), MgSO₄ (0.24 g/l), MnCl₂ (0.025 g/l) and FeSO₄ (0.015 g/l), lacking carbon and energy sources. The substrate 2-methylnaphtalene was added at a concentration of 50 µg/ml, using DMSO as solvent. Incubation was carried out at 35 ºC and shaking at 200 rpm for a reaction time of ten days. After this period, cells were harvested and menadione was extracted from the broth using dichloromethane. The product was concentrated by evaporation and analyzed by gas chromatography-mass spectrometry (GC-MS). The results showed a production of 1.3 mg of menadione per liter of broth.

### Morphological and biochemical characterization of Bacillus cereus biotype I

The characterization of the Bacillus cereus used in many of the examples was made by means of "Bergey's Manual of Systematic Bacteriology" (Sneath, Mair, Sharpe and Holt, 1986, Williams and Wilkins, eds., vol. 2). The ability to ferment different carbohydrates was determined by the use of the API 50 CHB system (BioMérieux). The bacterium was an endospore forming grampositive rod, with a central and elliptical spore which did not distinctly distend the sporangium. The spore broke the cell in an equatorial position. It grew at 45 ºC but not at 5 ºC or 55 ºC. It increased pH in Bacillus cereus agar and it presented lecitinase. As carbon sources, it oxidized and fermented the products D-glucose, D-fructose, N-acetylglucosamine, esculine, maltose, saccarose, trehalose, starch and xylitol. From all these properties, the Bacillus cereus used in many of the examples was characterized as a Bacillus cereus biotype I.

### Assay 2: Preparation of menadione with whole cells of Bacillus cereus biotype I

Preliminary growth of the microorganism: Precultures of Bacillus cereus biotype I were grown at pH 7 in trypticase soy medium. One percent of the culture was passed into minimal medium containing yeast extract (0.4 g/l), Na₂HPO₄ (4.4 g/l), KH₂PO₄ (2.45 g/l), (NH₄)₂SO₄(1.98 g/l), MgSO₄ (0.24 g/l), MnCl₂ (0.025 g/l), FeSO₄ (0.015 g/l) and sodium succinate (5.5 g/l). It was incubated at 35 ºC with vigorous shaking (200 rpm) until early stationary phase. Cells were harvested using a centrifugation step of 20 min at 4500 rpm, and they were washed in phosphate buffer.

Menadione preparation: Cells were passed into minimal medium containing yeast extract (0.4 g/l), Na₂HPO₄ (4.4 g/l), KH₂PO₄ (2.45 g/I), (NH₄)₂SO₄ (1.98 g/l), MgSO₄ (0.24 g/l), MnCl₂ (0.025 g/I) and FeSO₄ (0.015 g/l), lacking carbon and energy sources. The substrate 2-methylnaphtalene was added at a concentration of 50 µg/ml, using DMSO as solvent. Incubation was performed at 35 ºC and shaking at 200 rpm for a reaction time of ten days. After this period, cells were harvested and menadione was extracted from the broth using dichloromethane. The product was concentrated by evaporation and analyzed by GC-MS. The results showed a production of 1.5 mg of menadione per liter of broth.

### Assay 3: Preparation of menadione with disrupted cells of Bacillus cereus biotype I

Preliminary growth of the microorganism: This step was carried out as in Assay 2.

Cell disruption and menadione preparation: Cells were disrupted by lysozyme in five freeze-thaw cycles. Cell extracts were introduced into a solution containing KH₂PO₄ (50 mM), NADH (0.2 mM) and 2-methyl-1-naphtol (25 µg/ml) using DMSO as solvent, at 35 ºC, agitation at 200 rpm for five days. After this period, cells were harvested and menadione was extracted from the broth using dichloromethane. The product was concentrated by evaporation and analyzed by GC-MS. The results showed a production of 8 mg of menadione per liter of broth.

### Exploration assays with other culture media

The preparation of menadione was assayed with other culture media, shown in TABLE 1. Experiments were made with whole and disrupted cells, without preliminary growth.

Assays with whole cells: Bacillus cereus biotype I cells were introduced into the chosen medium, adding substrate in DMSO as solvent. Some assays were performed adding the organic solvent hexadecane to the used culture medium, from 25 mg/l to 5 g/l, in order to maximize the addition of substrate without increasing its toxicity. Incubation was performed at 35 ºC and agitation at 200 rpm for a reaction time of ten days. After this period, cells were harvested and menadione was extracted from the broth using dichloromethane. The product was concentrated by evaporation, and analyzed by GC-MS.

Assays with disrupted cells: Bacillus cereus biotype I cells were disrupted by lysozyme in five freeze-thaw cycles. Cell extracts were introduced into the selected solution and selected substrate, using DMSO as solvent, at 35 ºC and agitation at 200 rpm for five days. Some assays were performed adding the organic solvent hexadecane to the used culture medium, from 25 mg/l to 5 g/I, in order to maximize the addition of substrate without increasing its toxicity. After five days, cells were harvested and menadione was extracted from the broth using dichloromethane. The product was concentrated by evaporation and analyzed by gas GC-MS.

**TABLE 1. Some preparation assays of menadione**

| type of cells | culture medium components (g/l) | substrate (50 µg/ml) | addition of hexadecane (100 ml/l) | production of menadione |
|---|---|---|---|---|
| whole | minimal medium containing: yeast extract (0.4), Na₂HPO₄ (4.4), KH₂PO₄ (2.45), (NH₄)₂SO₄ (1.98), MgSO₄ (0.24), MnCl₂ (0.025), FeSO₄ (0.015), glucose (5.5) | 2-methyl-1-naphtol | no | yes |
| | | | yes | yes |
| whole | rich medium containing: soy peptone (5), casein peptone (15), sodium chloride (5) | 2-methyl-1-naphtol | no | yes |
| | | | yes | yes |
| whole | rich medium containing: peptone (10), Lab-lemco powder (10), sodium chloride (5) | 2-methyl-1-naphtol | no | yes |
| | | | yes | yes |
| disrupted | solution containing: KH₂PO₄ (50 mM), NADH (0.2 mM) | 2-methyl-naphtalene | no | yes |
| | | | yes | yes |

## Claims

1. A preparation process of menadione (I) comprising cultivating bacteria Bacillus cereus in a suitable culture medium comprising one or more substrates selected from the group consisting of 2-methylnaphtalene, 2-methyl-1-naphtol, and 3-methyl-1-naphtol.

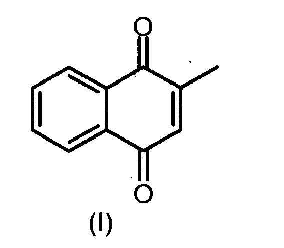

2. The process according to claim 1, wherein the culture medium comprises 2-methylnaphtalene, 2-methyl-1-naphtol or mixtures thereof.

3. The process according to claim 2, wherein the culture medium comprises 2-methylnaphtalene.

4. The process according to any of the claims 1-3, wherein the bacteria Bacillus cereus are of biotype I.

5. The process according to any of the claims 1-4, wherein the bacterial cells are disrupted.

6. The process according to any of the claims 1-4, wherein the bacterial cells are whole.

7. The process according to any of the claims 1-6, wherein the bacterial cells have been precultivated in a culture medium comprising a succinate alkaline salt.

8. The process according to claim 7, wherein the culture medium comprises a linear saturated hydrocarbon of 10 to 18 carbon atoms.

9. The process according to claim 8, wherein the hydrocarbon is hexadecane.

10. The process according to claim 9, wherein the concentration of hexadecane in the culture medium is from 25 mg/l to 5 g/l.

## Patentansprüche

1. Verfahren zur Herstellung von Menadion (I), bei dem Bacillus cereus-Bakterien in einem geeigneten Nährmedium kultiviert werden, das ein oder mehrere Substrate enthält, die aus der Gruppe bestehend aus 2-Methylnaphthalen, 2-Methyl-1-Naphthol und 3-Methyl-1-Naphthol ausgewählt wird/werden.

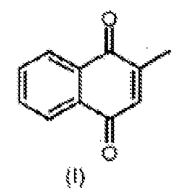

2. Verfahren nach Anspruch 1, bei dem das Nährmedium 2-Methylnaphthalen, 2-Methyl-1-Naphthol oder Mischungen davon enthält.

3. Verfahren nach Anspruch 2, bei dem das Nährmedium 2-Methylnaphthalen enthält.

4. Verfahren nach einem der Ansprüche 1-3, bei dem die Bacillus cereus-Bakterien vom Biotyp I sind.

5. Verfahren nach einem der Ansprüche 1-4, bei dem die Bakterienzellen zerstört sind.

6. Verfahren nach einem der Ansprüche 1-4, bei dem die Bakterienzellen ganz sind.

7. Verfahren nach einem der Ansprüche 1-6, bei dem die Bakterienzellen in einem Nährmedium vorkultiviert worden sind, das ein bernsteinsaures Alkalisalz enthält.

8. Verfahren nach Anspruch 7, bei dem das Nährmedium einen unverzweigten gesättigten Kohlenwasserstoff mit 10 bis 18 Kohlenstoffatomen umfasst.

9. Verfahren nach Anspruch 8, bei dem der Kohlenwasserstoff Hexadecan ist.

10. Verfahren nach Anspruch 9, bei dem die Hexadecankonzentration im Nährmedium zwischen einschließlich 25 mg/l und 5 g/l liegt.

## Revendications

1. Procédé de préparation de ménadione (I) comprenant la culture de bactéries Bacillus cereus dans un milieu de culture approprié comprenant un ou plusieurs substrats choisi/choisis dans le groupe constitué de 2-méthylnaphtalène, 2-méthyl-1-naphtol et 3-méthyl-1-naphtol.

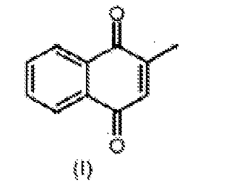

2. Procédé selon la revendication 1, dans lequel le milieu de culture comprend du 2-méthylnaphtalène, du 2-méthyl-1-naphtol ou leurs mélanges.

3. Procédé selon la revendication 2, dans lequel le milieu de culture comprend du 2-méthylnaphtalène.

4. Procédé selon l'une quelconque des revendications 1-3, dans lequel les bactéries Bacillus cereus sont de biotype I.

5. Procédé selon l'une quelconque des revendications 1-4, dans lequel les cellules bactériennes sont rompues.

6. Procédé selon l'une quelconque des revendications 1-4, dans lequel les cellules bactériennes sont entières.

7. Procédé selon l'une quelconque des revendications 1-6, dans lequel les cellules bactériennes ont été préalablement cultivées dans un milieu de culture comprenant un sel alcalin de succinate.

8. Procédé selon la revendication 7, dans lequel le milieu de culture comprend un hydrocarbure linéaire saturé de 10 à 18 atomes de carbone.

9. Procédé selon la revendication 8, dans lequel l'hydrocarbure est de l'hexadécane.

10. Procédé selon la revendication 9, dans lequel la concentration d'hexadécane dans le milieu de culture va de 25 mg/L à 5 g/L.
